# EUROPÄISCHE PATENTANMELDUNG

(11) **EP 2 832 846 A2**
(43) Veröffentlichungstag der Anmeldung: **04.02.2015**
(21) Anmeldenummer: 14002644.4
(22) Anmeldetag: 29.07.2014
(51) Int. Cl.: C12M 1/107, C12M 1/00, C12M 1/34, C12M 1/36

(54) **Biogasanlage mit geregeltem Gastransfer zwischen Gasspeichern**

(30) Priorität: 31.07.2013 DE 102013012707; 09.04.2014 DE 102014005231
(71) Anmelder: Seitz Electric GmbH, 86637 Wertingen/Bliensbach (DE)
(72) Erfinder: Seitz, Helmut, 86637 Wertingen (Bliensbach) (DE)

(57) **Zusammenfassung**

In einer Biogasanlage sind mehrere Gasspeicher beliebig miteinander vernetzt. Damit für einen angeschlossenen Gasverbraucher mit stark veränderlichem Gasverbauch ausreichende Versorgung gewährleistet ist, wird für die einzelnen Gasspeicher durch Gastransfer zwischen ihnen für ausgeglichene Füllstände gesorgt. Dazu wird aus allen Füllständen ein Füllstandsmittelwert errechnet, der als Sollwert für jeden einzelnen Gasspeicher dient.

## Beschreibung

Die Erfindung betrifft eine Biogasanlage mit mindestens zwei für einen Gastransfer durch Gasleitungen beliebig miteinander verbundenen Gasspeichern, deren Füllstände mittels Erfassungsvorrichtungen ermittelt und einer Regeleinrichtung zugeführt werden.

In EP 2346982 B1 sind Biogasanlagen mit einer Mehrzahl miteinander verbundener Biogasspeicher beschrieben. Mittels Druck- und/oder Füllstandserfassung und der entsprechenden Auswertung in einer Regeleinrichtung werden Gebläse bzw. Verdichter angesteuert um einen Gastransfer zwischen den Speichervolumina zu bewirken. Dabei kann bei Tragluftspeichern ein Gastransport durch Erzeugen von Differenzdrücken mittels Beeinflussung der Tragluftdrücke hergestellt werden. Alternativ bzw. ergänzend ist vorgesehen, den Gastransport durch von der Regeleinrichtung angesteuerte Verdichter in den Verbindungsrohren zu bewirken.

Wird das in einer Biogasanlage erzeugte Gas beispielsweise zur Speisung von Blockheizkraftwerken in der Stromversorgung verwendet, ist es wünschenswert, diese dem aktuellen Strombedarf entsprechend, also beispielsweise verstärkt in Spitzenlastzeiten, einzusetzen. Diese Betriebsart stellt hohe dynamische Anforderungen an die Gasversorgung bzw. das Gasmanagement. Bei kaskadierten oder netzartig verbundenen Gasspeichern muss dann für einen ausreichenden Durchsatz bzw. Gastransfer zwischen den bzw. durch die Gasspeicher hin zum Verbraucher gesorgt werden.

Dabei besteht das Problem, dass sich sehr unterschiedliche Füllstände einstellen können. Wünschenswert ist es aber, möglichst ausgeglichene Füllstände zu erreichen, um z. B. bei veränderlichen äußeren Einflüssen (Sonnenstrahlung, Wolkenabschattung) unerwünschte Drucksituationen zu vermeiden (unnötiges Ansprechen von Überdruckventilen, Unterdruck).

Bei einer Biogasanlage gemäß DE 102011051135 A1 wird hierfür in einem Hauptgasspeicher der Füllstand erfasst. Dieser dient als Sollwert für die Füllstände in zugeordneten Nebengasspeichern, die als Tragluftspeicher ausgeführt sind. Eine Regeleinrichtung, der alle Füllstände zugeführt werden, steuert Gebläse an, die den Trag- bzw. Stützluftdruck der Nebengasspeicher derart beeinflussen, dass die Füllstände dem Füllstand des Hauptgasspeichers angeglichen werden.

Der Erfindung liegt die Aufgabe zugrunde, eine für veränderlichen Gasverbrauch geeignete Biogasanlage zur Verfügung zu stellen.

In diesem Sinn verkörpert die vorliegende Erfindung eine einfache Lösung, die einen ausreichend schnellen Gastransfer zwischen den einzelnen Speichern sowie zum Verbraucher, z. B. einem Blockheizkraftwerk, sicherstellt. Dabei wird mittels einer Regeleinrichtung aus den Füllständen aller an der Füllstandsregelung beteiligten Gasspeichern ein ständig aktualisierter Mittelwert errechnet, der als Sollwert für den Füllstand eines jeden einzelnen Gasspeichers dient. Mittels der Regeleinrichtung werden Verdichter, Gebläse, Klappen und/oder Ventile derart angesteuert, dass durch Gastransfer zwischen den durch Gasleitungen beliebig miteinander verbundenen in die Regelung einbezogenen Gasspeichern Füllstände angestrebt werden, die dem als Sollwert dienenden Mittelwert entsprechen.

Die Gasspeicher können sich in oder auf den Gärbehältern befinden oder eigene Behälter sein. Es können Druckbehälter, beispielsweise Doppelmembran-Tragluftbehälter sein oder drucklose Behälter, beispielsweise ein Gassack. Die Speichervolumina können über Gasleitungen, z. B. Rohre beliebig miteinander verbunden sein, beispielsweise sternförmig oder kaskadenartig.

Dabei können für den Gastransfer Verdichter oder Gebläse in den Verbindungsrohren angeordnet sein, die von der Regeleinrichtung in geeigneter Weise angesteuert werden. Bei Tragluftspeichern kann durch Beeinflussung des Tragluftdruckes mittels Gebläsen eine für einen Gastransfer geeignete Druckdifferenz erzeugt werden. Bei vorhandener Druckdifferenz können von der Regeleinrichtung auch Klappen oder Ventile im Sinne der Angleichung der Füllstände an den Sollwert in geeigneter Weise angesteuert werden.

Die erfindungsgemäße Lösung für ein dynamisches Gasmanagement ist besonders vorteilhaft in größeren Biogasanlagen oder bei Nachrüstung oder Erweiterung solcher Anlagen. Denn es können unterschiedliche Varianten des Gastransfers leicht miteinander kombiniert werden. Entscheidend bleibt, dass die Füllstände der in das Gasmanagement einbezogenen Gasspeicher an der Bildung des Mittelwertes als Sollwert beteiligt werden. Die Herausnahme eines oder mehrerer Gasspeicher aus der Gasführung oder der Regelung hat keinen Einfluss auf das Regelverhalten der verbleibenden Anlage.

Vorteilhaft werden die Gebläse im Zweipunktmodus betrieben. Ein einstellbarer Totbandbereich verhindert zu kurze Ein- bzw. Aus-Zustände der Stützluftgebläse also eine Art "Flattern" und gewährleistet eine schonende Betriebsweise.

Vorteilhaft lässt sich das Prinzip der Erfindung bei bestehenden Biogasanlagen einsetzen, weil keine aufwändigen Maßnahmen erforderlich sind. Die Anzahl der Gasspeicher ist mindestens zwei und im Übrigen beliebig. Die Einstellung eines Offsets zum gemeinsamen Sollwert ermöglicht bei einzelnen Gasspeichern eine Betriebsweise mit vorgebbaren unterschiedlichen Füllständen. Durch Gewichtung der Größe der einzelnen Gasspeicher und Berücksichtigung der Form der Membranen (z.B. Halbkugel oder Kegel) mittels einer Konditionierung der Werte der erfassten Füllstände kann eine weitere Verbesserung des Gasmanagements erreicht werden.

Vorteilhafte Ausgestaltungen sind den weiteren Ansprüchen entnehmbar.

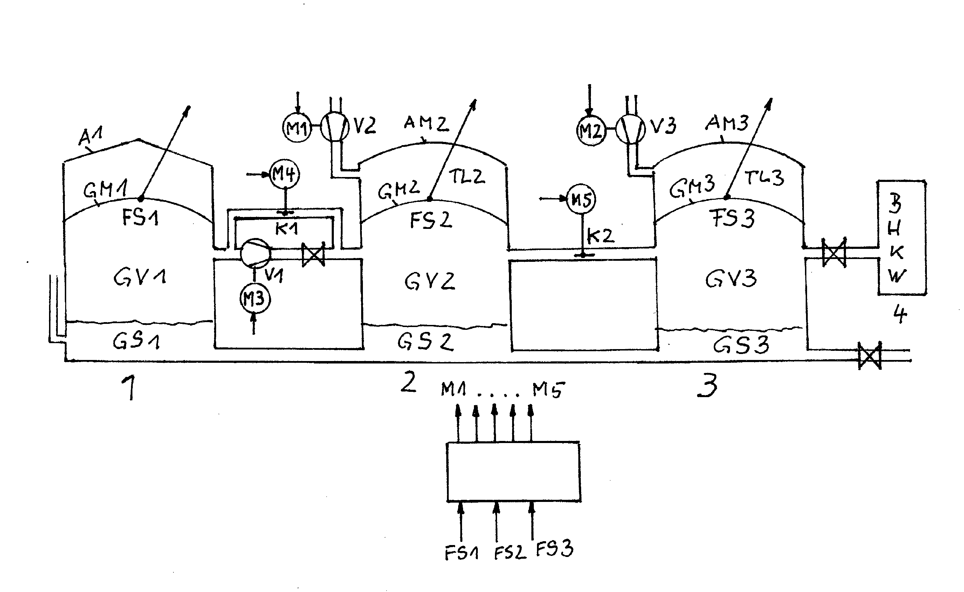

Ein prinzipielles auf die erfindungswesentlichen Merkmale beschränktes Ausführungsbeispiel wird nachstehend anhand der Zeichnung erläutert.

Die beispielhafte Biogasanlage besteht aus drei kaskadenartig miteinander verbundenen Behältern (1,2,3). Der erste Behälter (1) ist ein Fermenter, in den das frische Gärsubstrat (GS1) eingebracht ist. Über dem Gärsubstrat (GS1) befindet sich ein (Bio-) Gasvolumen (GV1), das durch eine Gasmembran (GM1) gasdicht abgedeckt ist. Der Behälter (1) ist durch eine gegen klimatische Einflüsse schützende Abdeckung (A1) abgeschlossen.

Ein zweiter Behälter (2) dient als Nachgärer, in dem sich das Gärsubstrat (GS2) befindet, das ihm über das Substratverbindungsrohr (SVR1) zugeführt wird. Über dem Gärsubstrat (GS2) befindet sich ein Gasvolumen (GV2) das durch eine Doppelmembran-Tragluftabdeckung gasdicht abgeschlossen ist. Dabei befindet sich zwischen einer das Biogasvolumen (GV2) abschließenden Gasmembran (GM2) und einer gasdichten Außenmembran (AM2) ein Tragluftvolumen (TL2). Zur Druckerzeugung im Tragluftvolumen (TL2) dient ein Verdichter oder Gebläse (V2) mit zugehörigem Antriebsmotor (M1). Die beiden Gasvolumina (GV1,GV2) sind über eine rohrförmige Gasleitung (GL1) miteinander verbunden. Im Zuge dieser Gasleitung ist ein Verdichter (V1) mit zugehörigem Antriebsmotor (M3) sowie ein Rückschlagventil angeordnet. Zum Verdichter (V1) und Rückschlagventil ist eine Gasleitung mit steuerbarer Klappe (K1) mit zugehörigem Antriebsmotor (M4) als Bypass parallel gelegt.

Ein dritter Behälter (3) ist gleichartig aufgebaut wie der zweite Behälter (2). Er dient als Endlager für das Gärsubstrat (GS3) und ist mit dem zweiten Behälter über ein Substratverbindungsrohr (SVR2) und eine Gasleitung (GL2) verbunden. Über eine weitere Gasleitung (GL3) besteht eine Gasverbindung mit einem Blockheizkraftwerk (BHKW).

Für den Betrieb der Biogasanlage ist eine Regeleinrichtung (RE) vorgesehen. Diese erhält Füllstandsignale von in allen Behältern (1,2,3) auf den Gasmembranen (GM1,GM2,GM3) angeordneten Füllstandssensoren (FS1,FS2,FS3). Aus den Füllstandssignalen errechnet die Regeleinrichtung (RE) ständig aktualisierend einen Füllstandsmittelwert. Dieser Mittelwert dient als Füllstand-Sollwert für jedes einzelne Gasvolumen (GV1,GV2,GV3). Die Regeleinrichtung erzeugt Steuersignale für Antriebsmotoren (M1,M2,M3,M4,M5). Der Motor (M3), der den Verdichter (V1) in der Gasleitung zwischen dem ersten und zweiten Behälter (1,2) antreibt, wird angesteuert, um einen Gastransfer vom ersten Behälter (1) zum zweiten Behälter (2) zu bewirken, falls z.B. der Füllstand im ersten Behälter höher als der Sollwert ist. Ein entgegengesetzter Gastransfer wird ermöglicht durch die vom Motor (M4) angetriebene Klappe (K1), falls die Sollwertvorgabe dies erfordert. Für den Gastransfer zwischen dem zweiten und dem dritten Behälter (2,3) erhalten die Motoren (M1,M2) der Tragluftgebläse (V2,V3) Steuersigale von der Regeleinrichtung (RE) zur Erzeugung eines derartigen Differenzdruckes in den Behältern, dass ein Füllstandsangleich an den Füllstandssollwert bewirkt wird. Aus dem dritten Behälter (3) wird Biogas über die Gasleitung (GL3) nach Bedarf zum Blockheizkraftwerk (BHKW) transferiert.

Bei der erfindungsgemäßen Biogasanlage wird das Blockheizkraftwerk (BHKW) in der erforderlichen Weise mit Biogas gespeist. Daneben findet aber zwischen den Behältern (1,2,3) ein bei Bedarf auch bidirektionaler Gastransfer statt, mit dem Ziel die Füllstände in den einzelnen Behältern immer dem gemeinsamen Mittelwert anzunähern. Mit dieser Betriebsweise ist auch bei stark veränderlichem Gasverbrauch durch das Blockheizkraftwerk immer ausreichend Biogas im dritten Behälter verfügbar, wobei gleichzeitig die Stabilität der Tragluftmembranen ohne zu großem Einsatz der Tragluftgebläse gewährleistet ist.

### Bezugszeichenliste

- 1, 2, 3:: Behälter mit Gasspeicher
- 4:: Blockheizkraftwerk, BHKW
- FS1, FS2, FS3:: Füllstandssensor, Erfassungsvorrichtung
- M1-M5:: (Antriebs- bzw. Stell-) Motor
- V1-V3:: Verdichter, Gebläse
- K1,K2:: steuerbare Klappe
- GV1-GV3:: Gasvolumen
- TL2,TL3:: Tragluftvolumen
- GS1-GS3:: Gärsubstrat
- SVR1-SVR3:: Substratverbindungsrohr
- GL1-GL3:: Gasleitung
- A1:: Abdeckung
- AM2,AM3:: Außenmembran
- GM1-GM3:: Gasmembran
- RE:: Regeleinrichtung

## Patentansprüche

1. Biogasanlage
- mit mindestens zwei für einen Gastransfer durch Gasleitungen (GL1,GL2) beliebig miteinander verbundenen Behältern mit Gasspeichern (1,2,3)
- deren Füllstand mittels Erfassungsvorrichtungen (FS1,FS2,FS3) ermittelt und einer Regeleinrichtung (RE) zugeführt wird,
- wobei die Regeleinrichtung (RE) aus sämtlichen Füllständen ständig aktualisierend einen Mittelwert errechnet, der als Sollwert für die Füllstände dient,
- wobei die Regeleinrichtung (RE) Steuersignale für zur Biogasanlage gehörende Verdichter, Gebläse, Klappen oder Ventile (V1,V2,V3,K1,K2) erzeugt, um einen geregelten Gastransfer zwischen den Gasspeichern (1,2,3) zu bewirken,
- und die Signale zur Ansteuerung der Verdichter, Gebläse, Klappen oder Ventile (V1,V2,V3,K1,K2) derart bemessen werden, dass ein Angleichen der Füllstände an den Sollwert erfolgt.

2. Biogasanlage nach Anspruch 1, **dadurch gekennzeichnet, dass** die einzelnen Füllstände im Verhältnis zum jeweiligen Gasvolumen (GV1,GV2,GV3) verarbeitet werden.

3. Biogasanlage nach Anspruch 1, **dadurch gekennzeichnet, dass** die Füllstände in der Regeleinrichtung (RE) für die Mittelwertberechnung als Prozentwerte, Höhenwerte oder als Volumenwerte verarbeitet werden.

4. Biogasanlage nach einem der vorangehenden Ansprüche, **dadurch gekennzeichnet, dass** für einzelne Gasspeicher (1,2,3) die Regelung auf einen um einen einstellbaren Offset verschobenen Sollwert erfolgt.

5. Biogasanlage nach einem der vorangehenden Ansprüche, **dadurch gekennzeichnet, dass** bei Gasspeichern in Doppelmembran-Tragluftspeicherausführung (1,2) der Tragluftdruck mittels von der Regeleinrichtung (RE) angesteuerten Tragluftgebläsen (V2,V3) derart eingestellt wird, dass ein Gastransfer im Sinne der Annäherung an den Füllstands-Sollwert durch Druckdifferenz bewirkt wird.

6. Biogasanlage nach einem der vorangehenden Ansprüche, **dadurch gekennzeichnet, dass** der Gastransfer durch Verdichter (V1) in den Gasleitungen (GL1) bewirkt wird.

7. Biogasanlage nach einem der vorangehenden Ansprüche, **dadurch gekennzeichnet, dass** bei durch Druckdifferenz bewirktem Gastransfer von der Regeleinrichtung (RE) Klappen (K1,K2) oder Ventile angesteuert werden.

8. Biogasanlage nach einem der vorangehenden Ansprüche, wobei die Verdichter (V1) und Tragluftgebläse (V2,V3) im Zweipunktmodus betrieben werden.

9. Biogasanlage nach einem der vorangehenden Ansprüche, wobei bei dem Zweipunktmodus ein einstellbares Totband vorgesehen ist.

10. Verfahren zum Betreiben einer Biogasanlage mit mindestens zwei für einen Gastransfer durch Gasleitungen (GL1,G12) beliebig miteinander verbundenen Gasspeichern (1,2,3) mit den Verfahrensschritten:
- Messen der Füllstände in den Gasspeichern und Zuführten der Füllstandsmesswerte zu einer Regeleinrichtung (RE)
- Ständig aktualisierendes Errechnen eines Füllstandsmittelwertes aus den Füllstandsmesswerten als Sollwert für alle Füllstände mittels der Regeleinrichtung (RE),
- Berechnen und Erzeugen von Steuersignalen mittels der Regeleinrichtung (RE) zur Ansteuerung von Verdichtern (V1), Gebläsen (V2,V3), Klappen (K1,K2) oder Ventilen derart, dass sich die Füllstände der einzelnen Gasspeicher (1,2,3) durch Gastransfer zwischen den Gasspeichern dem Sollwert annähern.
